# EUROPEAN PATENT SPECIFICATION

(11) **EP 0 947 162 B1**
(45) Date of publication and mention of the grant of the patent: **12.05.2004**
(21) Application number: 99200175.0
(22) Date of filing: 22.11.1993
(51) Int. Cl.: A61B 8/06, A61B 8/12, H01R 12/38

(54) **TERMINATION ASSEMBLY**
MONTAGESATZ FÜR LEITUNGSENDEN
ENSEMBLE DE CONNECTION POUR EXTREMITE DE CONDUCTEUR

(30) Priority: 23.11.1992 GB 9224585
(43) Date of publication of application: 06.10.1999
(62) Divisional of application: 93309307.2
(73) Proprietor: ADVANCED TECHNOLOGY LABORATORIES, INC., Bothell, Washington 98041 (US)
(72) Inventor: Nordgren, Timothy F., Bothell, Washington 98021 (US); Imling, Deborah K., A-5 Bellevue, Washington 98008 (US); Ungari, Joseph L., Everett, Washington 98206 (US); Killam, Donald Gilbert, Woodinville, Washington 98072 (US); McKeighen, Ronald E., Phoenix, AZ 85048 (US)
(74) Representative: Lottin, Claudine

(56) References cited:
- EP-A- 0 213 859
- EP-A- 0 366 405

## Description

This invention relates to a termination assembly for ultrasonic diagnostic probes or scanheads and, in particular, to ultrasound probes which are uniquely suited for imaging and diagnosing organs of the body and arterial vessels during surgery.

Ultrasonic diagnostic imaging probes, which generally find application during noninvasive procedures, can also be used during surgical procedures. One such procedure where ultrasonic imaging probes have particular utility is during vascular surgery. During vascular surgery, ultrasonic imaging probes can be utilized to image and diagnose the interior of carotid arteries. Another procedure where ultrasonic imaging probes have utility is during transplant surgery where, for example, the ultrasonic imaging probe can be used to verify successful attachment and function of renal arteries.

Surgical ultrasound probes are preferably small and as easily to manipulate as surgical instruments. Such probes should be capable of imaging the blood flow of a vessel which is in immediate contact with the probe. This necessitates focal properties of the probe which enable it to produce a focused image of tissue and structure which is in immediate contact with the probe. Reliability is enhanced by the provision of an all electronic probe with no mechanically moving parts. When using surgical probes it is important that the hand of the physician not obscure the surgical site being examined. Such probes must be adapted for use in a sterile surgical field, must pose no electrical hazard to the patient, and must be easy to clean and sterilize, e.g., should be smoothly and securely integrated and closed with no gaps or openings. Furthermore, such probes should be capable of use around organs and under skin which has not been incised during the surgical procedure.

Document EP-A-0 366 405 discloses a viabond tabcircuit electrical connector comprising a flexible planar body of nonconductive material having first and second apertures located therein and a plurality of conductive lines traversing said first aperture.

In accordance with the present invention there is provided a termination assembly as claimed in claim 1.

The transducer is preferably a piezoelectric, electronically steered and focused multielement transducer with no moving parts. The transducer end of the probe is preferably contained within an encapsulating boot and lens material, which provides reliable electronic insulating properties and is easy to clean and sterilize. The encapsulating boot also performs the function of a standoff, with the multielement transducer being capable of focusing at the tissue which is in immediate contact with the boot. The angulation of the handle permits the physician to grasp and manipulate the probe without blocking the physician's view of the surgical site. The offset attachment of the transducer end and shaft of the probe enables the physician to insert the distal tip of the transducer end under skin which has not been incised to examine tissues and arterial vessels peripheral to the point of incision.

In the drawings:
FIGURE 1 illustrates the outline of an intraoperative ultrasound probe;
FIGURE 2 illustrates a side view of the interior of the intraoperative ultrasound probe of FIGURE 1;
FIGURES 2a-2d illustrate various bottom, end and cross-sectional views of the probe of FIGURE 2;
FIGURE 3 is a partially cutaway view of the distal end of the probe of FIGURE 2;
FIGURE 4, 5, and 6 illustrate the manner in which the cable is attached to an intraoperative ultrasound probe of the present invention;
FIGURES 7a-7e illustrate the construction of a preferred termination assembly for connecting a multifilament coaxial cable to the intraoperative ultrasound probe; and
FIGURE 8 illustrates the fully assembled termination assembly of FIGURES 7a-7e.

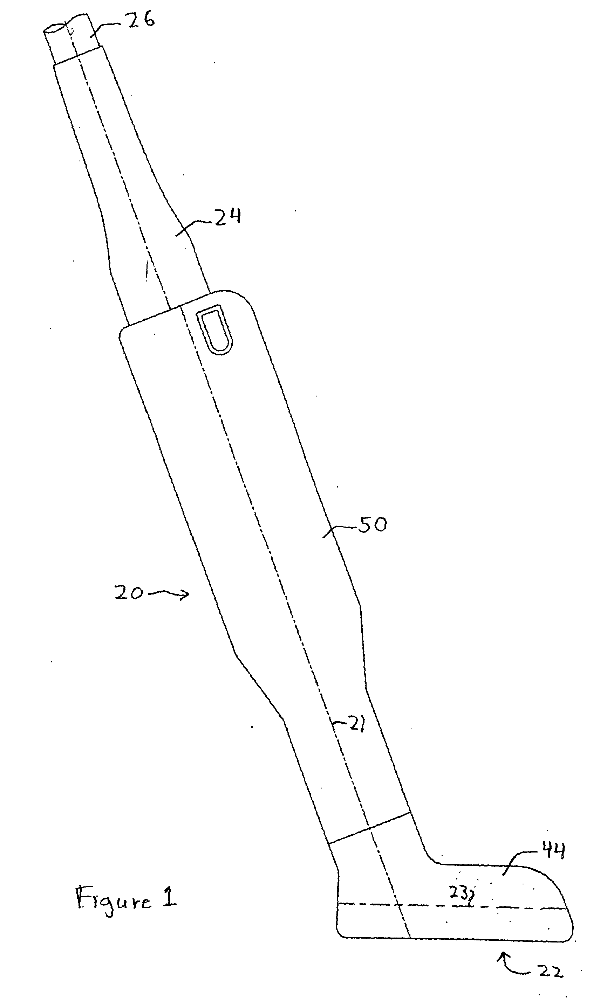

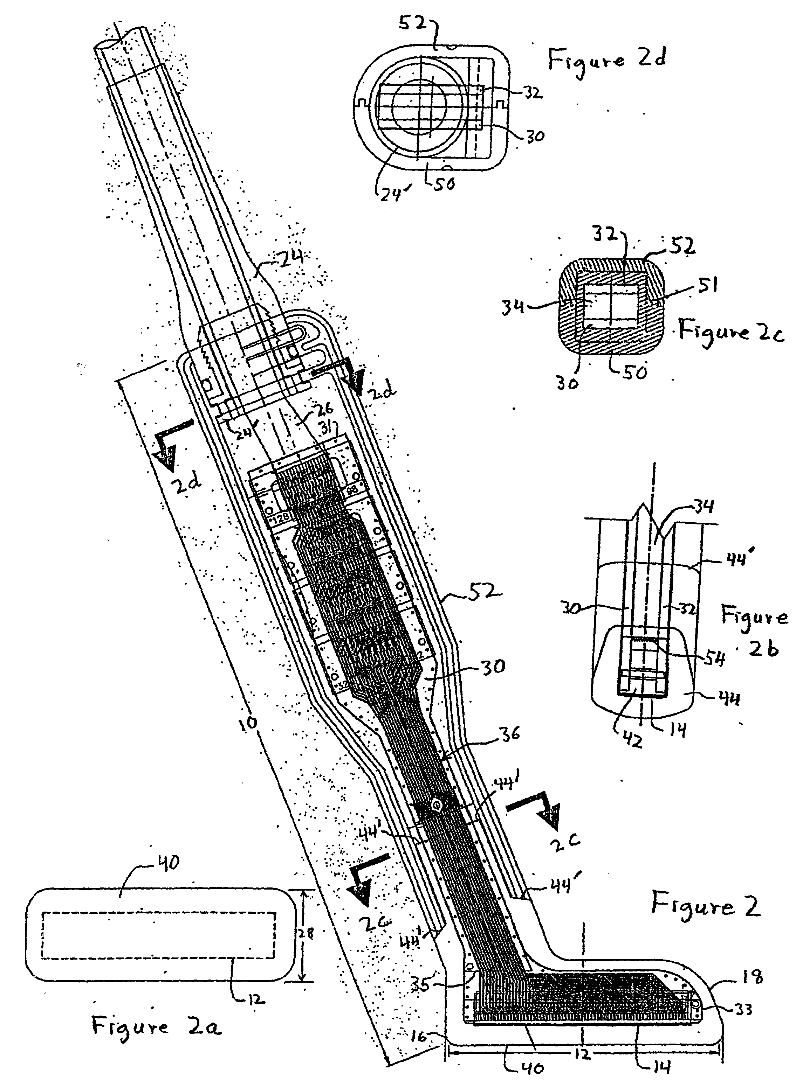

Referring first to FIGURES 1 and 2, an intraoperative ultrasound probe specifically designed for vascular surgical applications is shown. The probe is approximately L-shaped, resembling a tiny model of a leg and foot. The probe comprises a distal transducer section 22 resembling a boot on the "foot" and a handle section 20 resembling the "leg", with a strain relief 24 and multifilament cable 26 exiting the proximal end of the handle section 20. The cable leads to a connector (not shown) suitable for connecting the probe to an ultrasonic diagnostic system which drives the transducer of the probe and receives ultrasonic echo signals from the transducer in the conventional manner. A case with halves 50 and 52 form the exterior of the handle section 20 and the transducer section boot 44 is formed of molded rubber. The major axis 23 of the transducer section 22 is inclined at an angle of 112° with respect to the major axis 21 of the handle section. The length 10 of the handle section 20 from its proximal end to the heel 16 of the transducer section 22 is approximately 95 mm. The length 12 of the transducer section from heel 16 to its toe 18 is approximately 34.5 mm and the width 28 of the transducer section (FIGURE 2a) is approximately 12 mm.

Referring now to FIGURES 2 and 2a-2d, two matched printed circuit boards 30 and 32 are located inside the case 50, 52 and boot 44, each board having the inclined L-shape of the probe. Each printed circuit board extends from a top end 31 to a bottom distal end 33. The printed circuit boards are mounted in parallel, separated as shown in FIGURES 2b-2d by an insulating spacer 34, extending from the top 31 of the printed circuit boards to a lower terminating edge 35. Each printed circuit board contains a plurality of conductive traces 36, which make electrical connections between the transducer crystal 14 and the upper surfaces of the boards where connections are made to wires 38 of the multifilament cable 26.

The transducer crystal 14 is mounted perpendicular to the lower edges of the printed circuit boards 30 and 32 as shown in FIGURES 2, 2a, and 2b. The crystal 14 has an overall length of approximately 27 mm and a width of approximately 4.6 mm. The crystal 14 is diced into an array of 128 finely pitched elements for the performance of B-scan and color flow Doppler imaging. The finely pitched elements transmit and receive over an operating frequency range of 5-10 MHz, giving the transducer the ability to visualize and diagnose a vessel which is immediately in contact with the footprint 40 of the probe. The footprint of the probe is shown in the bottom view of FIGURE 2a, with the outline 12 of the transducer crystal indicated by a dashed line box.

The transducer crystal 14 is plated on its upper and lower surfaces with layers of conductive foil, one of which is cut by the dicing process. A plurality of metallic TAB leads are bonded at one end to the diced conductive foil with the other end of each TAB lead being bonded to a conductive trace 36 at the edges of the printed circuit boards 30 and 32. TAB lead connections are made to both the ground (undiced) and signal (diced) side of each piezoelectric element of the crystal and thence to the printed circuit boards 30 and 32.

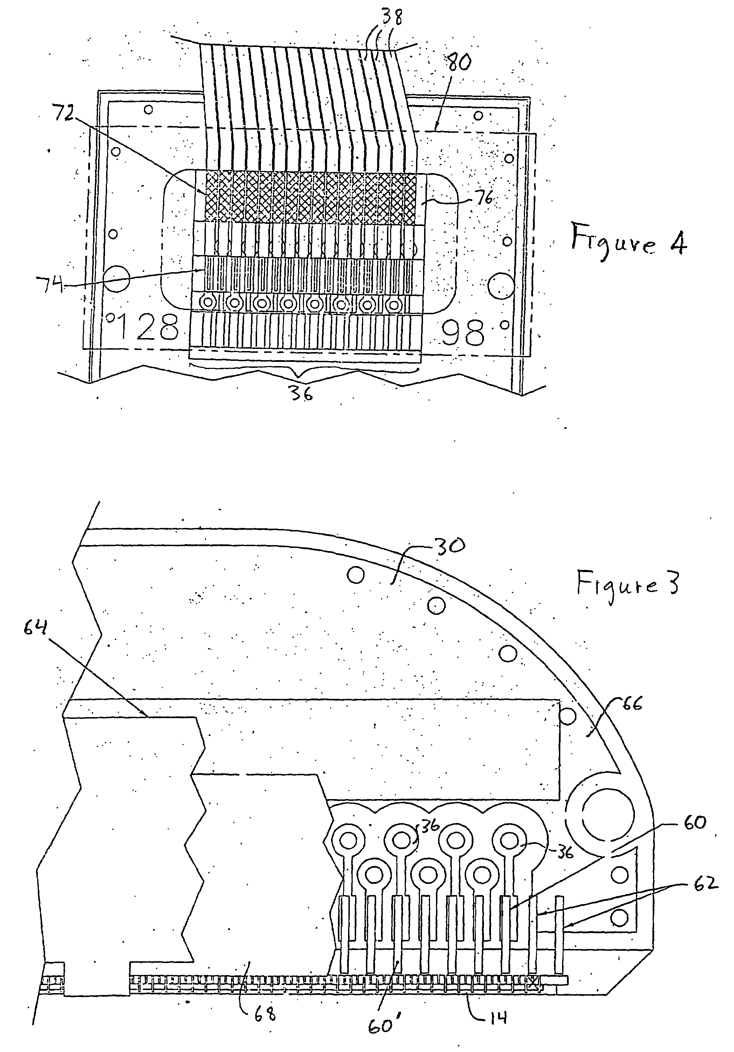

Turning to FIGURE 3, the manner in which electrical connections are made between the elements of the transducer crystal 14 and the conductive traces 36 of the printed circuit boards 30 and 32 is shown in enlarged detail. TAB leads 60, 60' are soldered at one end to the conductive foil strips on the inner surface of the transducer crystal 14. These strips have been formed, as mentioned above, by the dicing process. Two TAB leads 62 are connected to foil strips of inactive elements at the end of the transducer array. The other ends of the TAB leads 60 and 60' are soldered to conductive traces 36 on the printed circuit board 30. The ends of TAB leads 62 are soldered to the ground plane 66 of the printed circuit board.

A ground foil 64 folds over the outer foil surface of the transducer crystal and is attached to that foil surface to ground it. The ground foil 64 is also electrically connected to the ground plane 66 of the printed circuit board. A sheet 68 of nonconductive material insulates the conductive traces 36 from the ground foil 64.

It will also be noted in FIGURE 3 that TAB leads are only connected to alternating transducer elements. In this preferred embodiment, electrical connection is made to odd numbered elements from printed circuit board 32, and to even numbered elements from printed circuit board 30.

Returning to FIGURE 2 and 2a-2e, the volume 42 behind the transducer crystal and below the terminating edge 35 of the spacer 34, and between the printed circuit boards, is filled with a loaded epoxy backing material to a depth as indicated by the jagged upper surface 54 of the backing material in FIGURE 2b. The backing material attenuates acoustic waves emanating from the rear of the crystal. The entire transducer section is then encased in an RTV rubber compound by a molding process which forms the boot 44. As FIGURES 2 and 2b show, the RTV rubber casing 44 is approximately 2.5 mm thick below the transducer 14. This gives the probe an inherent standoff distance between the crystal and the vascular structure of the body that is in contact with the footprint 40 of the probe. The standoff distance allows vessels in contact with the footprint to be clearly imaged, as the standoff distance enables the return of ultrasonic echoes from the contacted vessel to return to a plurality of transducer elements for focusing in flow and image processing.

The RTV rubber casing 44 extends to edges 44' along the handle section of the probe and inside the case halves 50 and 52. If desired, the molding process can also form an integral liquid tight gasket 45 in the rubber where the lower portion of the case closes around the boot (see FIGURE 5). The encapsulating boot thus completely seals the transducer end of the probe, fully insulating the patient from the electrical connections to the transducer crystal. The encapsulating boot also renders the probe easy to clean and sterilize between uses, further enhancing patient safety and medical efficiency.

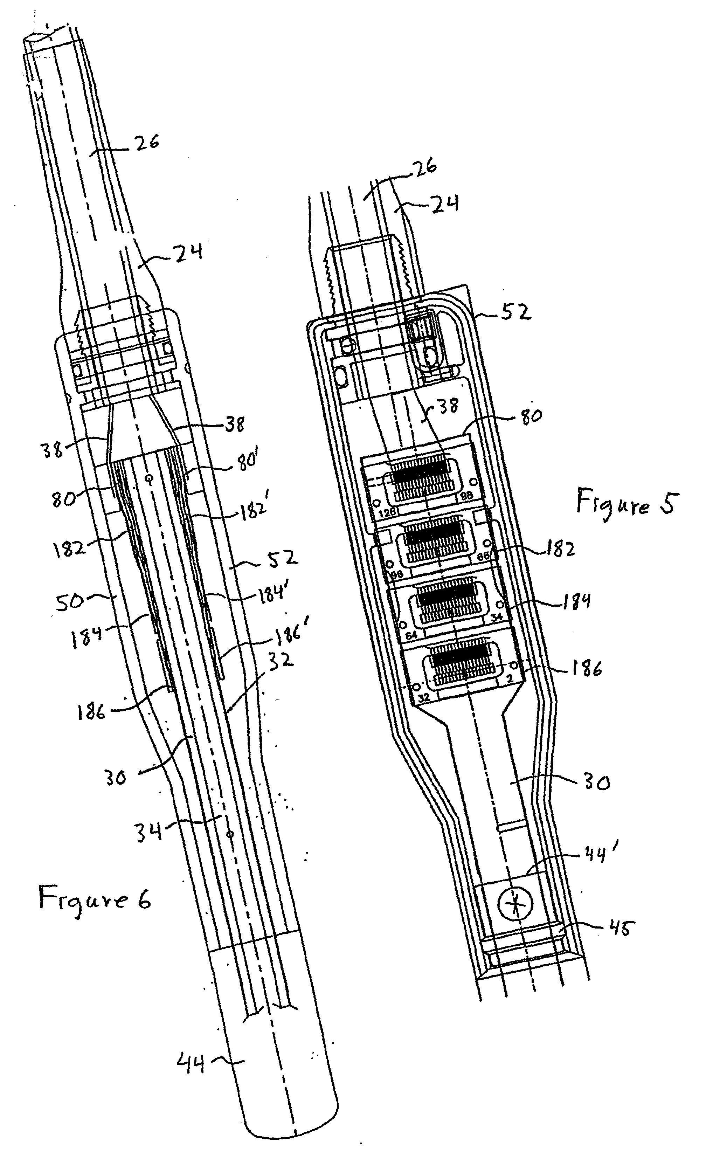

The handle section 20 above the edges 44' of the RTV rubber casing 44 comprises two hollow clamshell case halves 50 and 52 formed of a rigid polysulfone plastic. The case 50, 52 encloses the upper extension of the printed circuit boards 30 and 32 where connections are made to to the wires 38 of the cable 26. One technique for attaching the wires 38 of the cable 26 to the printed circuit boards 30 and 32 is shown in FIGURE 4, 5, and 6.

The preferred cable is comprised of 128 individual coaxial conductors. In FIGURE 4 reference numeral 38 designates the outer sheaths of several of these coaxial conductors. The outer sheaths terminate to expose the braided ground shields 72 of the coax, and these ground shields are soldered to a grounding pad 76 on the printed circuit board. The ground shields terminate and following a short length of inside insulating sheath the center conductors 74 of the coax are exposed. These center conductors are soldered to individual conductive signal traces 36 on the printed circuit board.

As FIGURE 5 shows, the coax wires are connected to the boards in groups of sixteen as denoted by terminating assemblies 80, 182, 184, and 186. Each terminating assembly terminates at a different distance along the cable so that the wires of each terminating assembly overlay the previous assemblies when attached to their positions on the printed circuit boards. This overlap is shown in FIGURE 6. In correspondence with the electrical connections to the transducer elements, odd numbered wires are connected to one of the printed circuit boards, and even numbered wires are connected to the other board. For ease of illustration of the termination assemblies, the overlapping wires have not been shown in FIGURE 5 but are illustrated in the profile view of FIGURE 6.

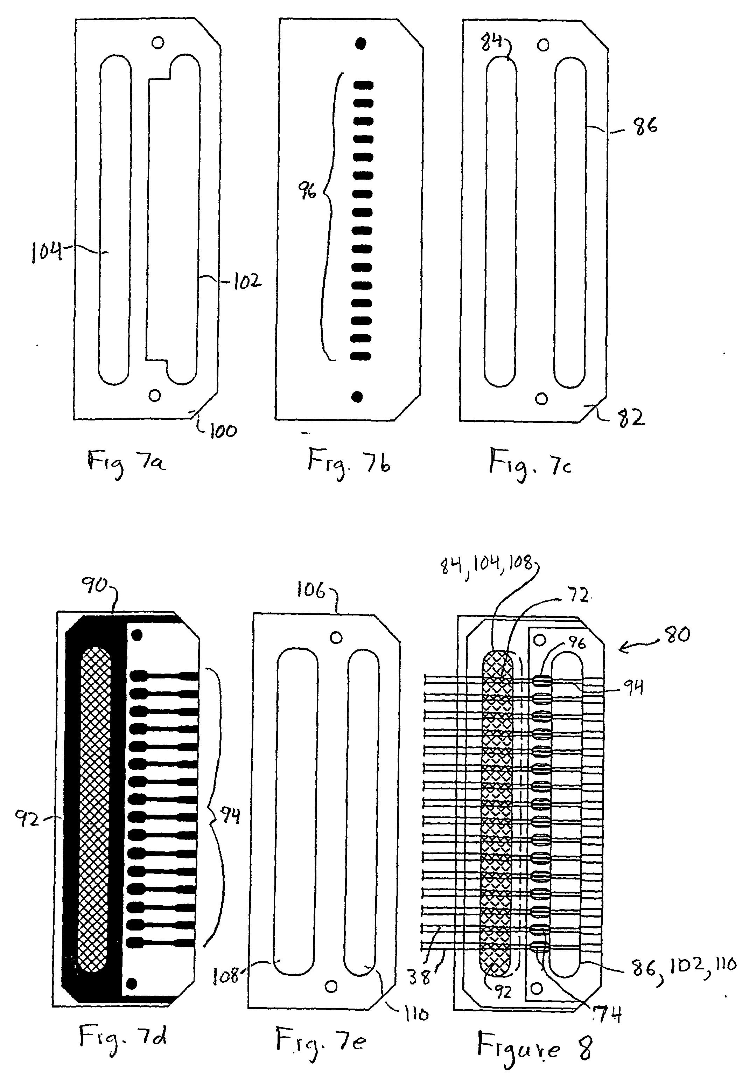

Preferably the cables are preassembled with the conductors of the coaxial wires soldered to terminating assemblies. The terminating assemblies are then easily soldered in position on the printed circuit boards 30 and 32. A preferred terminating assembly is shown in FIGURES 7a-7e and 8. Each terminating assembly is a multilayered panel of copper foil patterns and insulating sheets made of a flexible, insulating polyimide material. The terminating assembly includes a center polyimide sheet 82 as shown in FIGURE 7c. Photoetched on one side of the center sheet 82 is a line 96 of sixteen foil pads. Photoetched on the other side of the center sheet is a ground plane 90 with a central screen-like foil pattern 92, and sixteen bridging lines 94. Electrical through hole connections known as vias connect each pad 96 to a bridging line 94 on the other side of the center sheet 82. Apertures 84 and 86 are then formed in the center sheet 82, resulting in bridging lines 94 bridging the aperture 86 and the screen-like pattern 92 extending across aperture 84.

The assembly is completed by laminating an upper polyimide sheet 100 to the top of the center sheet, with aperture 102 exposing pads 96 and bridging lines 94, and aperture 104 exposing the copper screen 92. A lower polyimide sheet 106 with aligning apertures is laminated to the other side of the center sheet.

Each terminating assembly is attached to sixteen coaxial wires of the cable 26 as shown in FIGURE 8. The braided ground shields 72 are soldered to the screen-like foil pattern 92. During this process it is intended that solder will flow through the holes in the screen to the back side of the screen. The center coaxial conductors 74 are soldered to the individual foil pads 96 through aperture 102. Electrical connection is formed from the conductors 74 to the bridging lines 94 through the interconnecting vias.

Attachment of the cable to printed circuit boards 30 and 32 is now quick and easy. The screen-like foil pattern is positioned over the grounding pad 76 on the printed circuit board with the bridging lines 94 aligned with their mating conductive traces 36 on the board. In one operation the solder which flowed through to the back of the screen 92 is heated to attach the ground shields and screen to the pad 76. If an insufficient amount of solder flowed to the back side of the screen during the prior assembly step, this soldering step may be augmented by providing additional solder to the ground shields so that the shields and screen a securely soldered to the pad 76. A second operation simultaneously contacts bridging lines 94 through aperture 102 and solders the lines 94 to the underlying conductive traces 36. The soldering of the bridging lines should not loosen the conductors 74 from pads 96 due to the low thermal conductivity of the electrically interconnecting vias. In this manner the eight terminating assemblies 80, 80', 182-196 and 182'-186' are quickly attached to the printed circuit boards of the probe.

After all of these electrical connections have been made, the case 50, 52 is closed around the RTV rubber casing 44 at the distal end of the case and a flange 24' of the strain relief 24 at the proximal end of the case and sealed with an RTV adhesive to form a liquidtight enclosure, thereby enabling the probe to be safely and reliably used in a surgical field and thereafter sterilized.

As FIGURES 2c and 2d show, at the wider top of the handle the case is rounded in the back with squared corners in the front (FIGURE 2d), and makes a transition to its narrower, lower dimension where it is approximately square in cross-section (FIGURE 2c). This shape enables the surgeon to hold and handle the probe in the manner of a surgical instrument, with the rounded back resting between the thumb and forefinger and the squared corners affording a secure grip for the fingers.

The inclined L-shape of the probe enables the surgeon to grasp the handle section 20 and manipulate the probe without the hand obscuring the surgical site and vessel being contacted by the probe footprint 40. The transducer section 22 can be moved along a vessel to image the vessel even beyond the extent of an incision, because the toe 18 of the transducer section can be inserted under the skin at the end of the incision. This enables the surgeon to view and diagnose flow conditions in the vessel beyond the incision site.

## Claims

1. A termination assembly for attaching coaxial wires (38) of a multiwire cable (26) to conductive traces (36) of a printed circuit comprising:
a flexible planar body (82,100,106) of nonconductive material having first and second apertures (84,86) located therein;
a plurality of conductive lines (94) traversing said first aperture (86) and suitable for connection to individual ones of the central conductors (74) of said coaxial wires (38) and to conductive traces (36) of a printed circuit board; and
a sheet (92) of conductive material traversing said second aperture (84) and suitable for common connection to the shield conductors (72) of said coaxial wires (38) and to a ground plane (90) of said printed circuit board.

2. The termination assembly of claim 1, wherein said sheet (92) of conductive material further comprises a plurality of perforations permitting the flow of solder therethrough.

3. The termination assembly of claim 1 or claim 2, wherein said flexible planar body comprises first and second sheets (100,106) of nonconductive material each having aligning first and second apertures (102, 104, 108, 110),
wherein said conductive lines (94) and said sheet of conductive material (92) are located intermediate said first and second sheets, and
wherein said conductive lines (94) traverse said aligned, first apertures (102, 110), and
wherein said sheet (92) of conductive material traverses said aligned second apertures (104, 108).

4. The termination assembly of claim 3, further comprising a third sheet (82) of nonconductive material having first and second apertures (84,86) aligning with the first and second apertures of said first and second sheets,
wherein said conductive lines and said sheet of conductive material are affixed to said third sheet, and
wherein said third sheet is laminated between said first and second sheets.

5. The termination assembly of any preceding claim, further comprising a plurality of conductive pads (96) aligned with said conductive lines and electrically connected to said conductive lines,
wherein said conductive pads are suitable for connection to individual ones of the central conductors of said coaxial wires.

6. The termination assembly of claim 5, wherein said conductive pads (96) are relatively thermally isolated from said conductive lines.

7. The termination assembly of claim 5 or claim 6, wherein said conductive pads (96) are located on a different planar surface of said flexible body than said conductive lines.

8. The termination assembly of any one of claims 5 to 7, wherein said conductive pads (96) are electrically connected to said conductive lines by vias.

## Patentansprüche

1. Abschlußbaugruppe zum Befestigen von koaxialen Drähten (38) eines Mehrdraht-Kabels (26) an Leiterbahnen (36) einer gedruckten Schaltung mit:
einem flexiblen planaren Körper (82, 100, 106) aus nichtleitendem Material mit einer ersten und einer zweiten darin liegenden Öffnung (84, 86);
einer Vielzahl von leitfähigen Leitungen (94), die die genannte erste Öffnung (86) durchqueren und zum Anschluss an einzelne der mittleren Leiter (74) der genannten koaxialen Drähte (38) und an Leiterbahnen (36) einer Leiterplatte geeignet sind; und
einer Lage (92) aus leitfähigem Material, die die genannte zweite Öffnung (84) durchquert und zum gemeinsamen Anschluss an die Abschirmleiter (72) der genannten koaxialen Drähte (38) und an eine Masse-Ebene (90) der genannten Leiterplatte geeignet ist.

2. Abschlußbaugruppe nach Anspruch 1, wobei die genannte Lage (92) aus leitfähigem Material weiterhin eine Vielzahl aus Perforationen umfasst, die zulassen, daß das Lötmittel hindurchfließt.

3. Abschlußbaugruppe nach Anspruch 1 oder Anspruch 2, wobei der genannte flexible planare Körper eine erste und eine zweite Lage (100, 106) aus nichtleitendem Material umfasst, die je eine ausrichtende erste und zweite Öffnung (102, 104, 108, 110) aufweisen,
wobei die genannten leitfähigen Leitungen (94) und die genannte Lage aus leitfähigem Material (92) zwischen der genannten ersten und zweiten Lage liegen und
wobei die genannten leitfähigen Leitungen (94) die genannten ausgerichteten, ersten Öffnungen (102, 110) durchqueren und
wobei die genannte Lage (92) aus leitfähigem Material die genannten ausgerichteten zweiten Öffnungen durchquert (104, 108).

4. Abschlußbaugruppe nach Anspruch 3, weiterhin mit einer dritten Lage (82) aus nichtleitendem Material mit einer ersten und einer zweiten Öffnung (84,86), ausgerichtet zur ersten und zweiten Öffnung der genannten ersten und zweiten Lage,
wobei die genannten leitfähigen Leitungen und die genannte Lage aus leitfähigem Material an der genannten dritten Lage befestigt sind, und
wobei die genannte dritte Lage zwischen die genannte erste und zweite Lage laminiert ist.

5. Abschlußbaugruppe nach einem der vorhergehenden Ansprüche, weiterhin mit einer Vielzahl von leitfähigen Lötflächen (96) die zu den genannten leitfähigen Leitungen ausgerichtet sind und mit den genannten leitfähigen Leitungen elektrisch verbunden sind,
wobei die genannten leitfähigen Lötflächen zum Anschluss an einzelne der mittleren Leiter der genannten koaxialen Drähte geeignet sind.

6. Abschlußbaugruppe nach Anspruch 5, wobei die genannten leitfähigen Lötflächen (96) von den genannten leitfähigen Leitungen relativ wärme-isoliert sind.

7. Abschlußbaugruppe nach Anspruch 5 oder 6, wobei die genannten leitfähigen Lötflächen (96) auf einer anderen planaren Oberfläche des genannten flexiblen Körpers liegen als die leitfähigen Leitungen.

8. Abschlußbaugruppe nach einem der Ansprüche 5 bis 7, wobei die genannten leitfähigen Lötflächen (96) mit den genannten leitfähigen Leitungen über Durchkontaktierungen elektrisch verbunden sind.

## Revendications

1. Ensemble d'extrémité pour rattacher des fils coaxiaux (38) d'un câble multifilaire (26) à des rubans conducteurs (36) d'un circuit imprimé comprenant :
un corps plan flexible (82, 100, 106) de matériau non conducteur comportant des première et deuxième ouvertures (84, 86) situées dans celui-ci ;
une pluralité de lignes conductrices (94) traversant ladite première ouverture (86) et appropriées pour une connexion à des uns individuels des conducteurs centraux (74) desdits fils coaxiaux (38) et à des rubans conducteurs (36) d'une carte à circuits imprimés, et
une feuille (92) de matériau conducteur traversant ladite deuxième ouverture (84) et appropriée pour une connexion commune aux conducteurs blindés (72) desdits fils coaxiaux (38) et à un plan de masse (90) de ladite carte à circuits imprimés.

2. Ensemble d'extrémité suivant la revendication 1, dans lequel ladite feuille (92) de matériau conducteur comprend en outre une pluralité de perforations permettant le flux de soudure à travers celles-ci.

3. Ensemble d'extrémité suivant la revendication 1 ou 2, dans lequel ledit corps plan flexible comprend des première et deuxième feuilles (100, 106) de matériau non conducteur comportant chacune des premières et deuxièmes ouvertures (102, 104, 108, 110) alignées,
dans lequel lesdites lignes conductrices (94) et ladite feuille de matériau conducteur (92) se trouvent entre lesdites première et deuxième feuilles, et
dans lequel lesdites lignes conductrices (94) traversent lesdites premières ouvertures (102, 110) alignées, et
dans lequel ladite feuille (92) de matériau conducteur traverse lesdites deuxièmes ouvertures (104, 108) alignées.

4. Ensemble d'extrémité suivant la revendication 3, comprenant en outre une troisième feuille (82) de matériau non conducteur comportant des première et deuxième ouvertures (84, 86) s'alignant avec les première et deuxième ouvertures desdites première et deuxième feuilles,
dans lequel lesdites lignes conductrices et ladite feuille de matériau conducteur sont fixées à ladite troisième feuille, et
dans lequel ladite troisième feuille est laminée entre lesdites première et deuxième feuilles.

5. Ensemble d'extrémité suivant l'une quelconque des revendications précédentes, comprenant en outre une pluralité de plages de connexion conductrices (96) alignées avec lesdites lignes conductrices et électriquement connectées auxdites lignes conductrices,
dans lequel lesdites plages de connexion conductrices sont appropriées pour une connexion à des uns individuels des conducteurs centraux desdits fils coaxiaux.

6. Ensemble d'extrémité suivant la revendication 5, dans lequel lesdites plages de connexion conductrices (96) sont relativement thermiquement isolées desdites lignes conductrices.

7. Ensemble d'extrémité suivant la revendication 5 ou 6, dans lequel lesdites plages de connexion conductrices (96) se trouvent sur une surface plane différente dudit corps flexible de celle desdites lignes conductrices.

8. Ensemble d'extrémité suivant l'une quelconque des revendications 5 à 7, dans lequel lesdites plages de connexion conductrices (96) sont électriquement connectées auxdites lignes conductrices par des traversées.
